# FASCICULE DE BREVET EUROPEEN

(11) **EP 3 685 596 B1**
(45) Date de publication et mention de la délivrance du brevet: **03.04.2024**
(21) Numéro de dépôt: 18803731.1
(22) Date de dépôt: 21.09.2018
(51) Int. Cl.: H04R 25/00, A61B 5/12, A61B 5/00, A61B 1/227

(54) **SYSTEME DE MESURE AUDIOMETRIQUE A DISTANCE ET PROCEDES ASSOCIES POUR L'ETABLISSEMENT D'UN PROFIL AUDITIF ET LE REGLAGE DES PROTHESES AUDITIVES A L'AIDE D'UN TEL SYSTEME**
FERNHÖRTESTSYSTEM UND ZUGEHÖRIGE VERFAHREN ZUR ERSTELLUNG EINES HÖRPROFILS UND ZUR EINSTELLUNG VON HÖRGERÄTEN MIT EINEM SOLCHEN SYSTEM
REMOTE HEARING TEST SYSTEM AND ASSOCIATED METHODS FOR ESTABLISHING AN AUDITORY PROFILE AND ADJUSTING HEARING AIDS USING SUCH A SYSTEM

(30) Priorité: 21.09.2017 FR 1758749
(43) Date de publication de la demande: 29.07.2020
(60) Demande divisionnaire: 24157590.1
(73) Titulaire: Neocustic, 06560 Valbonne (FR)
(72) Inventeur: CHENG, Kwok Kuen, 78100 Saint Germain En Laye (FR); CHENG, Kwok Wai, 94130 Nogent Sur Marne (FR); ROUVET, Laurent, 94130 Nogent Sur Marne (FR); DIDI, Thierry, 75015 Paris (FR)
(74) Mandataire: IP Trust
(86) Numéro de dépôt international: PCT/FR2018/052298
(87) Numéro de publication internationale: WO 2019/058061

(56) Documents cités:
- WO-A1-00/42816
- CN-A- 101 442 699
- DE-A1-102010 041 529
- US-A1- 2004 073 136
- US-A1- 2005 059 904
- US-A1- 2010 305 469
- US-A1- 2017 071 534
- MATTHEW A. BROMWICH ET AL: "Active Noise Reduction Audiometry: A Prospective Analysis of a New Approach to Noise Management in Audiometric Testing", THE LARYNGOSCOPE, vol. 118, no. 1, 1 janvier 2008 (2008-01-01), pages 104-109, XP055099710, ISSN: 0023-852X, DOI: 10.1097/MLG.0b013e31815743ac

## Description

### DOMAINE TECHNIQUE DE L'INVENTION

L'invention concerne le domaine de l'audiologie et plus particulièrement un système de mesure audiométrique à distance en vue de l'établissement d'un bilan auditif d'une personne ainsi que du réglage à distance des prothèses auditives en fonction du bilan auditif établi.

### ETAT DE LA TECHNIQUE

Classiquement, les tests auditifs sont réalisés par un professionnel de la santé, tel qu'un audioprothésiste, dans un environnement médical adapté. Les tests auditifs se déroulent conventionnellement en trois étapes. La première étape consiste en un interrogatoire de la personne concernée par le test auditif au sujet des symptômes auditifs, des antécédents médicaux, etc. La deuxième étape consiste en la réalisation d'un examen des conduits auditifs externes et des tympans des oreilles afin de vérifier l'état de ces derniers et de détecter d'éventuelles causes de la diminution de l'audition (otite, présence importante de cérumen, etc.). L'examen visuel des tympans est réalisé à l'aide d'un otoscope. La troisième étape consiste en la réalisation d'un audiogramme, et plus particulièrement d'une audiométrie tonale. Cet examen a pour but de déterminer les seuils auditifs pour chaque oreille. Il est réalisé dans une pièce ou cabine insonorisée, avec un audiomètre apte à produire des sons (fréquences pures) dans chacune des oreilles pour la mesure du seuil d'audition. L'opérateur situé à l'extérieur de la cabine pilote l'audiomètre pour mesurer les seuils auditifs de chaque oreille sur chacune des plages de fréquence, les seuils maximum (seuil d'inconfort) tolérés par la personne et la compréhension de la personne en lui faisant entendre et reconnaitre les mots.

Une fois le bilan auditif réalisé, l'audioprothésiste détermine si une aide auditive est requise, et dans l'affirmative, recommande des prothèses auditives qu'il convient de régler pour qu'elles soient adaptées au profil auditif de la personne. Le réglage consiste à exécuter un algorithme prenant en compte l'audiogramme de la personne effectué, mais aussi les données personnelles de la personne (âge, sexe, etc.). Les paramètres obtenus sont ensuite configurés directement sur la prothèse auditive. Un premier affinement du réglage en fonction du ressenti de la personne a lieu en suivant. Un deuxième affinement du réglage a lieu dans un deuxième temps, après quelques jours ou semaines de port de prothèses. Le porteur retourne donc chez l'audioprothésiste lequel effectue une vérification et un nouveau réglage si nécessaire.

De tels tests et réglages présentent cependant l'inconvénient d'être relativement couteux et restent relativement contraignants en terme de temps pour la personne passant les tests. A cela s'ajoute le fait que les personnes ayant une baisse ou une mauvaise audition choisissent de s'isoler plutôt que de consulter.

Afin de palier ces inconvénients, et d'inciter les personnes présentant des troubles d'audition à consulter un professionnel de l'audition, des méthodes d'évaluation en ligne ou par téléphone de la capacité auditive ont été développées. Ces méthodes ont été développées afin qu'une personne puisse obtenir, à titre indicatif, un premier bilan de sa capacité auditive sans se rendre dans une structure médicale. Citons à titre d'exemple la méthode décrite dans la demande de brevet US2010/0305469.

Ces méthodes ne sont cependant pas totalement satisfaisantes car elles ne prennent pas en compte le niveau sonore ambiant dans lequel les personnes réalisent le test auditif. Par ailleurs, les tests d'autoévaluation réalisés par un particulier, en ligne ou non, ne permettent pas un calibrage du matériel utilisé (casque et amplification) et de ce fait, ne garantit pas l'obtention d'un audiogramme fiable. En outre, elle ne résout pas le problème de réglage des prothèses. Bien souvent, les personnes s'en tiennent aux tests auditifs, ne poursuivant pas la démarche par l'achat et le réglage de prothèses auditives.

L'invention vise à remédier à ces problèmes en proposant un système de mesure audiométrique facilitant la réalisation d'audiogrammes et le réglage des prothèses auditives.

### OBJET DE L'INVENTION

A cet effet, et selon un premier aspect, l'invention propose un système de mesure audiométrique à distance comprenant un équipement de commande d'une prothèse auditive comportant une interface de communication arrangée pour permettre une communication bidirectionnelle, un logiciel pour exécuter des séquences de sons prédéfinies et enregistrées dans un module mémoire dudit équipement en réponse à une instruction en provenance d'une station de commande à distance équipée d'un logiciel de contrôle à distance, au moins une prothèse auditive comportant une interface de communication arrangée pour permettre une communication bidirectionnelle avec l'équipement de commande, ledit équipement comprenant une couche logicielle de communication avec la prothèse auditive d'une part et étant agencé pour réaliser une passerelle entre la station de commande laquelle est équipée d'un logiciel de contrôle à distance de la prothèse auditive et la prothèse auditive d'autre part, et des moyens d'isolation phonique de la prothèse auditive.

Grâce à cette architecture, les tests auditifs et le réglage des prothèses auditives en fonction des paramètres établis à partir des tests auditifs sont réalisés à distance, de manière simple, rapide et fiable.

Les séquences de sons prédéfinies et enregistrées dans le module mémoire dudit équipement sont exécutées avantageusement par l'équipement de commande en réponse à une instruction de commande effectuée directement depuis la station de commande. Il s'agit dans ce cas d'une exécution instantanée des séquences de sons. Il peut être également prévu, selon une autre mise en oeuvre, que les séquences de sons soient exécutées par l'équipement de commande en réponse à une instruction de commande effectuée directement via ledit équipement et après réception d'une instruction d'autorisation d'exécution en provenance de la station de commande. Il s'agit dans cette mise en oeuvre d'une exécution différée des séquences de sons.

Avantageusement, les séquences de sons prédéfinies et enregistrées dans le module mémoire sont également stockés dans un sous module de l'application mobile téléchargeable sur l'équipement de commande.

Selon l'invention, le système comporte en outre des moyens de prise d'image arrangés avec la prothèse pour permettre une visualisation de l'intérieur de l'oreille lorsque celle-ci est équipée de la prothèse auditive.

Avantageusement, la prothèse auditive comporte un corps de prothèse comprenant un transducteur d'entrée pour la réception d'un signal d'entrée et un microcontrôleur configuré pour commander le fonctionnement de la prothèse auditive 2, la prothèse auditive comportant en outre un transducteur de sortie électro-acoustique pour la production d'un signal de sortie perceptible sous la forme de signaux sonores et un conduit de transmission de signaux pour la transmission de signaux représentatifs des signaux sonores à produire par le transducteur de sortie électro-acoustique ou produit par ce dernier.

Selon un mode de réalisation particulier, le transducteur de sortie électro-acoustique est logé dans le corps de prothèse. Dans cette configuration, le conduit de transmission de signaux est un conduit acoustique guidant les sons produits par le transducteur de sortie. Selon une variante de réalisation, le transducteur de sortie électro-acoustique est déporté en extrémité du conduit de transmission de signaux, ce dernier constituant dans cette configuration un conduit électrique.

Avantageusement, les moyens de prise d'images sont ménagés en extrémité du conduit de transmission de signaux.

Avantageusement, le conduit de transmission de signaux est raccordé de manière amovible au corps de prothèse.

Avantageusement, le conduit amovible est apte à être connecté à une autre prothèse auditive, la couche logicielle de communication de l'équipement de commande pouvant être adaptée à ladite autre prothèse, de manière à élargir le périmètre d'utilisation de la solution.

Avantageusement, la prothèse auditive comporte des moyens de mesure de la pression sonore.

Avantageusement, les moyens d'isolation phonique se présentent sous la forme d'un casque ou plus avantageusement un casque actif pour éliminer les fréquences basses.

Avantageusement, le système de mesure audiométrique (application diagnostic à distance) est déclenché en mode Saas, et les données et résultats du bilan personnalisé du testeur sont stockées également en mode Saas afin d'en assurer la sécurité et confidentialité L'application mobile installée sur le smartphone (équipement de commande) exécute le programme de mesure audiométrique et les données et résultats sont transférés au serveur du système en fin de programme pour un stockage sécurisé et une analyse approfondie par des professionnels d'audiométrie.

L'invention concerne également un procédé pour établir un bilan auditif d'une personne à l'aide d'un système de mesure audiométrique à distance tel que décrit précédemment, la personne étant équipée des prothèses auditives aux fins de la réalisation d'un bilan auditif, lesdites prothèses étant associées à des moyens d'isolation phonique, caractérisé en ce qu'il comprend les étapes consistant à :
- transmettre des données relatives à des séquences de signaux sonores à la prothèse auditive par l'intermédiaire de l'équipement de commande,
- transmettre le contenu des réponses vocales de la personne à la station de commande par l'intermédiaire de l'équipement de commande,
- mesurer en continu la pression acoustique présente au sein de l'équipement d'isolation phonique et transmission des données à la station de commande par l'intermédiaire de l'équipement de commande.

Avantageusement, les données relatives à des séquences de signaux sonores sont transmises à la prothèse auditive soit depuis la station de commande par l'intermédiaire de l'équipement de commande, soit par l'équipement de commande en réponse à une autorisation de transmission en provenance de la station de commande.

L'invention concerne également un procédé pour le réglage d'une prothèse auditive comportant une interface de communication bidirectionnelle à l'aide d'un système de mesure audiométrique à distance tel que décrit précédemment, le procédé comprenant une étape de transmission des données de réglage de la prothèse à partir d'un profil auditif de la personne établi depuis la station de commande par l'intermédiaire de l'équipement de commande.

L'invention concerne également un procédé pour stimuler la capacité auditive d'une personne à l'aide d'un système de mesure audiométrique à distance tel que décrit précédemment à partir d'un bilan auditif établi pour la personne suivant le procédé d'établissement d'un bilan auditif d'une personne tel que décrit précédemment.

### BREVE DESCRIPTION DES FIGURES

D'autres objets et avantages de l'invention apparaîtront au cours de la description qui suit, faite en référence aux dessins annexés, dans lesquels :
- la figure 1 représente une vue synoptique d'un système de mesure audiométrique à distance ;
- la figure 2 représente un schéma fonctionnel de la prothèse auditive mise en oeuvre dans le système de la figure 1.

### DESCRIPTION DETAILLEE DES FIGURES

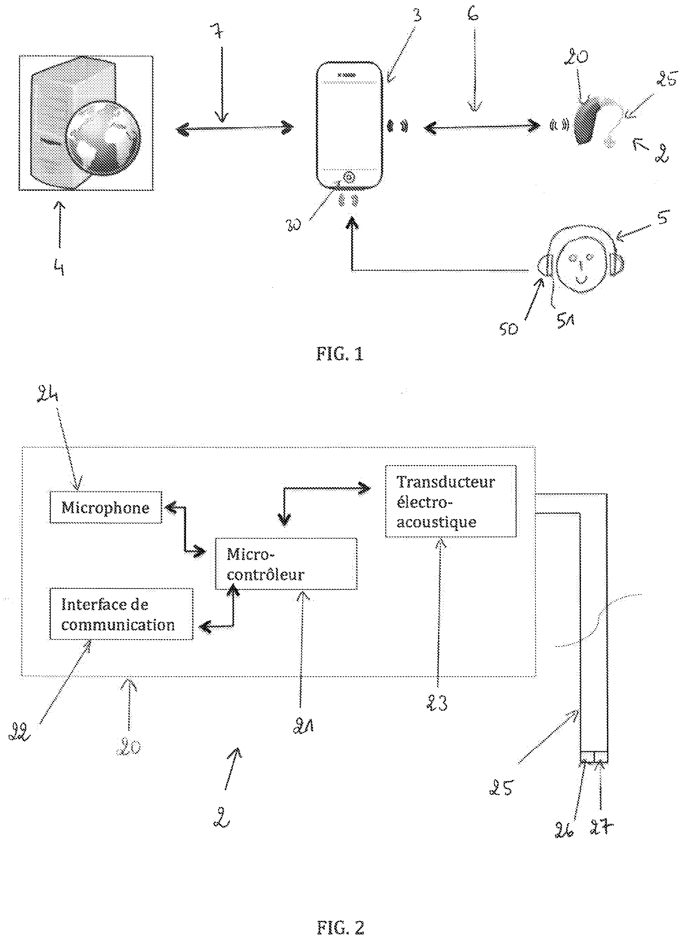

En relation avec la figure 1, il est décrit un système 1 de mesure audiométrique à distance. Comme on le comprendra plus loin, le système de mesure audiométrique à distance permet également de paramétrer à distance une ou des prothèse(s) auditive(s) en fonction du profil auditif établi à partir des mesures audiométriques relevées.

Le système 1 de mesure audiométrique comprend deux prothèses auditives 2 (une prothèse pour l'oreille droite et une prothèse pour l'oreille gauche - une seule prothèse étant représentée), un équipement de commande 3 de la prothèse auditive 2 et une station de commande 4 distante comprenant une base de données patients et un logiciel de contrôle à distance. La prothèse auditive 2 est connectée à l'équipement de commande 3 via une liaison sans fil du type Bluetooth, lui-même connecté à la station de commande 4 via un réseau de communication 7 du type Internet et réseau de télécommunication mobile.

L'équipement de commande 3, de préférence mobile, représenté sur la figure 1, est un téléphone intelligent (Smartphone). Il s'agit d'un exemple de réalisation, tout autre équipement de communication ou terminal, mobile ou non, tel qu'une tablette numérique ou similaire, constitue un équipement de commande 3 selon l'invention. Les caractéristiques décrites par la suite en relation avec le Smartphone s'appliquent donc également par extension à tout type d'équipement de communication ou terminal autre qu'un Smartphone apte à être mis en oeuvre selon la présente invention. Comme on le verra plus loin, l'équipement de commande 3 (Smartphone) sert de passerelle de communication, avec des moyens de contrôle d'accès aux prothèses, entre la station de commande 4 qui peut être le terminal distant de l'audioprothésiste et les prothèses auditives portées par la personne. Il comporte une application propriétaire qui sert de relais de communication entre la station de commande 4 et les prothèses, avec un contrôle d'accès aux prothèses, conçue pour configurer chacune des prothèses auditives 2 en fonction des instructions reçues depuis la station de commande.

La prothèse auditive 2 dont le schéma fonctionnel est illustré sur la figure 2 est une prothèse auditive connectée de format du type BTE (Behind The Ear) à contour d'oreille. Il pourrait bien entendu s'agir d'une prothèse de format RIC (Receiver in Canal) ou de format ITE (In the Ear) ou de tout autre format de prothèse disponible ou à venir sans sortir du cadre de l'invention.

Selon le format BTE, la prothèse 2 comporte un corps de prothèse 20 comprenant, outre un transducteur d'entrée pour la réception d'un signal sonore d'entrée (non représenté), un microcontrôleur 21 configuré pour commander le fonctionnement de la prothèse auditive 2, une interface de communication sans fil 22 (Bluetooth) configurée pour permettre une communication bidirectionnelle avec l'équipement de commande 3 lequel comporte également une interface de communication sans fil (Bluetooth).

Le corps de prothèse 20 comporte en outre un transducteur de sortie électro-acoustique 23 (haut-parleur) configuré pour produire des signaux sonores à des fréquences pures selon des séquences de sons prédéfinies et enregistrées dans un module mémoire de l'équipement de commande 3. Ainsi configurée, la prothèse auditive 2 joue le rôle, avec l'équipement de commande 3, d'audiomètre.

Le corps de prothèse 20 comporte également un moyen de mesure, tel qu'un microphone 24, pour mesurer le niveau sonore de l'environnement dans lequel les prothèses auditives sont situées. Le niveau sonore est mesuré dans les différences fréquences du spectre sonore à laquelle les oreilles humaines sont sensibles (fréquences comprises entre 16 Hz et 8000 Hz. Comme on le verra plus loin, les mesures relevées par le microphone 24 sont transmises à la station de commande 4 via l'équipement de commande 3 afin de vérifier si le niveau de bruit environnant à la prothèse est inférieur à un seuil limite au-delà duquel le niveau sonore est considéré comme non acceptable pour effectuer un test auditif.

Le corps de prothèse 20 est pourvu d'un conduit acoustique 25 permettant le guidage des sons produits par le haut parleur à l'intérieur de l'oreille dans laquelle l'extrémité du conduit acoustique est placée. Dans le cas d'une prothèse de format RIC, le transducteur de sortie électro-acoustique est intégré dans l'embout de la prothèse destinée à entrer dans le conduit auditif de la personne portant la prothèse auditive. Dans ce cas, le conduit reliant le transducteur de sortie à la partie électronique contenue dans le corps de boitier sera non pas un conduit acoustique mais un conduit électrique. Dans le cas d'une prothèse de format ITE, le transducteur de sortie électro-acoustique est complètement intégré dans la prothèse qui s'insère dans le conduit auditif après empreinte personnalisée, c'est une prothèse dite « invisible ».

Le système 1 de mesure audiométrique comporte en outre des moyens de prise d'images. Dans le mode de réalisation illustré, les moyens de prise d'image comportent une caméra 26 et des moyens d'éclairage 27 associés qui sont intégrés dans le conduit 25 de la prothèse auditive 2. Cela permet ainsi à l'opérateur de visualiser les tympans du patient depuis la station de commande 4 via le Smartphone 3 ou un autre équipement externe auquel la caméra est raccordée. Comme illustré sur la figure 2, la caméra est placée en extrémité du conduit 25. Il en est de même des moyens d'éclairage. Il peut être prévu également, selon une variante de réalisation, d'équiper la paroi extérieure du conduit 25 d'une ou plusieurs fibres optiques pour guider la lumière en provenance d'une source lumineuse située au niveau du corps de prothèse 20 ou à distance de celle-ci, la (les) fibres optique(s) s'étendant avantageusement jusqu'au niveau de l'extrémité proximale du conduit (extrémité la plus éloignée du corps 20 de prothèse). Il peut être prévu également de placer la caméra à l'extrémité de la fibre optique opposée à l'extrémité débouchant au niveau de l'extrémité proximale du conduit.

Avantageusement, le conduit 25 est raccordé de manière amovible au corps de prothèse 20. Cela permet ainsi d'interchanger les conduits, rendant ainsi la prothèse auditive 2 modulable. En particulier, cela permet de passer d'une prothèse instrumentée dite prothèse de test permettant de réaliser les mesures audiométriques aux fins d'établir un bilan auditif de la personne, à une prothèse d'aide à l'audition à proprement parler. Dans cette dernière configuration, le conduit acoustique 25 monté sur le corps de la prothèse serait un simple conduit acoustique dépourvu de caméra et de moyens d'éclairage associés.

Le système 1 de mesure comporte en outre des moyens d'isolation phonique configurés pour isoler d'un point de vue phonique la prothèse auditive 2 des bruits environnants. Avantageusement, les moyens d'isolation phonique se présentent sous la forme d'un casque 5 présentant sur la totalité du périmètre de chacune de ses coques 50 une couche de matériau isolant 51 (figure 1). Avantageusement, il peut être prévu que le casque 5 soit un casque actif permettant d'améliorer l'isolation phonique des basses fréquences. Par basse fréquence, on entend dans la présente demande, des fréquences inférieures ou égales à 1000 Hz. Il pourra être ainsi avantageusement utilisé la technologie ANR comme les casques de la gamme NoiseMaster^{™}. Comme on le verra plus loin, les moyens d'isolation phonique sont utilisés durant les tests auditifs, lors de la réalisation du bilan. Il est bien entendu évident que l'invention ne se limite pas à ce type d'équipement, et que tout autre équipement permettant d'isoler le patient des bruits environnements peut être mis en oeuvre sans sortir du cadre de l'invention.

Afin d'améliorer l'isolation acoustique au niveau des prothèses auditives 2, le conduit acoustique 25 peut comporter en extrémité un embout en mousse, avantageusement à usage unique.

Le bilan auditif et le paramétrage des prothèses auditives 2 en fonction du bilan auditif réalisé s'effectuent de la manière suivante.

Il est procédé en premier lieu à l'établissement du profil auditif de la personne. Pour ce faire, celle-ci s'équipe des prothèses auditives 2 et du casque 5 d'isolation phonique pour placer les prothèses dans un environnement adéquat, i.e. un environnement limité en termes de bruits environnants.

L'opérateur envoie depuis la station de commande 4 les instructions au Smartphone 3 pour activer l'application et le programme associé d'établissement du profil auditif. A réception des instructions, le Smartphone 3 transmet par Bluetooth les données enregistrées dans le module mémoire et relatives aux séquences de signaux sonores instruites pour la réalisation du test auditif à chacune des prothèses auditives 2 selon les instructions données par l'opérateur. Les données sont transmises simultanément ou successivement selon le programme lancé. Les données reçues par les prothèses auditives 2 sont ensuite traitées et transmises au transducteur 23 qui reproduit les séquences de signaux sonores à l'intérieur du canal auditif du porteur de prothèse via le conduit acoustique 25 de la prothèse. Les réponses vocales du porteur de prothèses à réception des signaux sonores sont récupérées par le microphone 30 du Smartphone pour être transmises à la station de commande 4 distante. Les réponses du porteur sont enregistrées dans un module mémoire de la station de commande 4 distante.

Concomitamment à la transmission des signaux sonores à produire par les prothèses auditives 2, le niveau sonore présent à l'intérieur du casque 5 (dans l'exemple illustré, à l'intérieur de chacune des coques du casque) est mesuré et transmis en temps réel au Smartphone 3 par Bluetooth qui le transmet via Internet à la station de commande distante. Les données relatives au niveau sonore mesurées durant le test auditif sont mémorisées dans le module mémoire de la station de commande 4.

Préalablement au lancement du test auditif, l'opérateur peut procéder à une vérification de l'intérieur des oreilles, et notamment vérifier que celles-ci ne comportent pas d'éléments susceptibles de causer une diminution de l'audition, comme par exemple la présence d'un bouchon de cérumen. Pour ce faire, l'opérateur active à distance la caméra 26 prévue à l'extrémité du conduit acoustique 25 des prothèses auditives 2 via le Smartphone 3 et vérifie en temps réel l'intérieur de l'oreille. Cette étape est facultative.

L'audioprothésiste établit le bilan auditif du patient à partir des réponses du patient reçues des prothèses auditives 2 via le Smartphone 3.

Il est procédé ensuite aux réglages des prothèses auditives 2 destinées à être portées à partir du bilan auditif établi. Ces réglages sont transmis à chacune des prothèses auditives 2 via le Smartphone 3. Plus spécifiquement, et comme pour la réalisation du test auditif, les réglages à effectuer à la ou les prothèses auditives 2 sont transmis à l'application dédiée téléchargée sur le Smartphone qui sert de passerelle de communication entre la station de commande 4 distante auquel l'opérateur se connecte et les prothèses auditives 2 du porteur. L'application configure la ou les deux prothèse(s) auditive(s) en fonction des données de réglages reçues.

Dans l'exemple qui vient d'être décrit, le bilan auditif est réalisé suite à une instruction de commande provenant directement de l'audioprothésiste depuis la station de commande. Il peut être prévu également selon une variante de mise en oeuvre que la réalisation d'un diagnostic à distance s'effectue suite à une instruction de commande de bilan auditif directement depuis le Smartphone. Dans ce cas, le porteur de prothèse lancera l'application dédiée pour la réalisation du diagnostic et préalablement installée sur le Smartphone qui déroulera les séquences de signaux sonores pour chacune des prothèses. L'application sera lancée après réception, le cas échéant, d'une autorisation d'exécution en provenance de la station de commande. Les réponses vocales ou tactiles du porteur en réponse aux signaux sonores émis sont ensuite enregistrées puis traitées via l'application dédiée du Smartphone. A l'issue du traitement des réponses du porteur de prothèses, la capacité auditive de chacune des oreilles est validée et un bilan auditif préliminaire du porteur établi. Ces résultats sont ensuite transmis à la station de commande pour l'établissement d'un bilan auditif complet et validé par l'audioprothésiste.

Avantageusement, l'application installée sur le smartphone est configurée pour permettre au porteur de prothèses d'intervenir sur l'amplification générale des réglages personnalisés préprogrammées, appelés réglages fins personnalisés. Il peut ainsi notamment intervenir sur le volume sonore émis par les prothèses et le moduler en plus grave ou plus aigu. Il peut également spécifier un environnement d'usage parmi un groupe d'environnements déterminés (télévision, restaurant bruyant, rue, conversation salon privé, etc.). L'environnement d'usage peut être choisi soit manuellement par le porteur de prothèse, soit automatiquement via un algorithme d'Intelligence Artificielle (IA).

Les données d'usage et les environnements d'usage sélectionnées (son amplifié, environnement choisi, ou autres) sont alors collectés et transmis dans une base de données personnelles du patient hébergée sur la station de commande et rendue accessible à l'audioprothésiste pour des analyses et optimisation des réglages fins personnalisés ultérieurs

Il peut être prévu également la mise en oeuvre du système de mesure audiométrique précédemment décrit pour stimuler la capacité auditive d'un porteur de prothèses (rééducation auditive). Pour ce faire, le Smartphone 3 (et plus généralement l'équipement de commande) comporte une application dédiée à cette mise en oeuvre. Pour activer la stimulation auditive, le porteur lance l'application dédiée laquelle envoie une séquence de signaux programmés pour stimuler l'attention du porteur en prenant en compte le niveau de handicap auditif. L'application enregistre alors les réactions du porteur ou les actions de ce dernier (réponses vocales ou tactiles) pendant le déroulement du programme. Les résultats obtenus sont ensuite transmis par l'application à la station de commande pour être comparés avec les résultats précédemment mémorisés à des programmes de stimulation et ainsi permettre la mesure des progrès de compréhension accomplis par le porteur. A partir des progrès relevés, un nouveau programme de stimulation tenant compte des progrès auditifs du porteur est établi pour poursuivre la progression dans la compréhension des mots et des sons et transmis au patient via son Smartphone.

Avantageusement, les séquences de sons prédéfinies dans le cadre du bilan auditif, celles pré-définies dans le cadre de la rééducation auditive et la partie IA dans le cadre de la sélection de l'environnement d'usage constituent des sous-modules indépendants hébergés dans une même application, chacun des sous-modules étant téléchargeables indépendamment les uns des autres sur le Smartphone (et plus généralement l'équipement de commande).

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Système (1) de mesure audiométrique à distance comprenant :
- un équipement de commande (3) d'une prothèse auditive (2) comportant une interface de communication arrangée pour permettre une communication bidirectionnelle et un logiciel pour exécuter des séquences de sons prédéfinies et enregistrées dans un module mémoire de l'équipement en réponse à une instruction en provenance d'une station de commande à distance (4),
- au moins une prothèse auditive (2) comportant une interface de communication (22) arrangée pour permettre une communication bidirectionnelle avec l'équipement de commande (3), ledit équipement (3) comprenant une couche logicielle de communication avec la prothèse auditive (2) d'une part et étant agencé pour réaliser une passerelle entre la station de commande (4) laquelle est équipée d'un logiciel de contrôle à distance de la prothèse auditive (2) et la prothèse auditive (2) d'autre part, et
- des moyens d'isolation phonique (5) de la prothèse auditive (2), **caractérisé en ce qu'**il comporte des moyens de prise d'images arrangés avec la prothèse pour permettre une visualisation de l'intérieur de l'oreille lorsque celle-ci est équipée de la prothèse auditive (2).

2. Système (1) de mesure audiométrique à distance selon la revendication 1, **caractérisé en ce que** la prothèse auditive (2) comporte un corps de prothèse (20) comprenant un transducteur d'entrée pour la réception d'un signal d'entrée et un microcontrôleur (21) configuré pour commander le fonctionnement de la prothèse auditive (2), cette dernière comportant en outre un transducteur de sortie électro-acoustique pour la production d'un signal de sortie perceptible sous la forme de sons et un conduit de transmission de signaux pour la transmission de signaux représentatifs du son à produire par le transducteur de sortie électro-acoustique ou en provenance de ce dernier.

3. Système (1) de mesure audiométrique à distance selon la revendication 2, **caractérisé en ce que** les moyens de prise d'images sont ménagés en extrémité du conduit de transmission de signaux.

4. Système (1) de mesure audiométrique à distance selon la revendication 2 ou la revendication 3, **caractérisé en ce que** le conduit de transmission de signaux est raccordé de manière amovible au corps de prothèse (20).

5. Système (1) de mesure audiométrique à distance selon l'une quelconque des revendications précédentes, **caractérisé en ce que** la prothèse auditive (2) comporte des moyens de mesure de la pression sonore.

6. Système (1) de mesure audiométrique à distance selon l'une quelconque des revendications précédentes, **caractérisé en ce que** les moyens d'isolation phonique se présentent sous la forme d'un casque de préférence actif.

7. Procédé pour établir un bilan auditif d'une personne à l'aide d'un système (1) de mesure audiométrique à distance selon l'une quelconque des revendications précédentes, la personne étant équipée des prothèses auditives aux fins de la réalisation d'un bilan auditif, lesdites prothèses étant associées à des moyens d'isolation phonique, **caractérisé en ce qu'**il comprend les étapes consistant à :
- transmettre des données relatives à des séquences de signaux sonores à la prothèse auditive par l'intermédiaire de l'équipement de commande,
- transmettre le contenu des réponses vocales de la personne à la station de commande par l'intermédiaire de l'équipement de commande,
- mesurer en continu la pression acoustique présente au sein de l'équipement d'isolation phonique et transmission des données à la station de commande par l'intermédiaire de l'équipement de commande.

8. Procédé pour établir un bilan auditif selon la revendication précédente, **caractérisé en ce que** les données relatives à des séquences de signaux sonores sont transmises depuis la station de commande à la prothèse auditive par l'intermédiaire de l'équipement de commande.

9. Procédé pour établir un bilan auditif selon la revendication 7, **caractérisé en ce que** les données relatives à des séquences de signaux sonores sont transmises à la prothèse auditive par l'équipement de commande en réponse à une autorisation de transmission depuis la station de commande.

10. Procédé pour le réglage d'une prothèse auditive (2) comportant une interface de communication bidirectionnelle à l'aide d'un système (1) de mesure audiométrique à distance selon les revendications 1 à 6, le procédé comprenant une étape de transmission des données de réglage de la prothèse à partir d'un profil auditif de la personne établi depuis la station de commande par l'intermédiaire de l'équipement de commande.

11. Procédé pour le réglage d'une prothèse auditive (2) selon la revendication 10, **caractérisé en ce qu'**il comporte une étape de sélection d'un environnement d'usage parmi un groupe d'environnement déterminés enregistrés dans un sous-module d'une application dédiée téléchargée sur l'équipement de commande.

12. Procédé pour le réglage d'une prothèse auditive (2) selon la revendication 11, **caractérisé en ce que** l'environnement d'usage est sélectionné à l'aide d'un algorithme d'Intelligence Artificielle en utilisation les données d'usage colletées de chaque porteur de prothèses.

13. Procédé pour le réglage d'une prothèse auditive (2) selon la revendication 11 ou la revendication 12, **caractérisé en ce que** les données d'usage et l'environnement d'usage sélectionné sont collectés et transmis dans une base de données personnelles du patient hébergée sur la station de commande (4) pour des analyses et une optimisation de réglages fins personnalisés ultérieurs.

14. Procédé pour stimuler la capacité auditive d'une personne à l'aide d'un système (1) de mesure audiométrique à distance selon l'une quelconque des revendications 1 à 6 comprenant une étape d'établissement d'un bilan auditif pour la personne suivant le procédé selon l'une quelconque des revendications 7 à 9.

## Patentansprüche

1. Fernhörtestsystem (1), umfassend:
- eine Steuereinrichtung (3) eines Hörgeräts (2), aufweisend eine Kommunikationsschnittstelle, die derart eingerichtet ist, dass eine bidirektionale Kommunikation erfolgen kann, und eine Software zum Ausführen vordefinierter Tonfolgen, die in einem Speichermodul der Einrichtung gespeichert sind, als Reaktion auf eine Anweisung von einer Fernsteuerungsstation (4),
- mindestens ein Hörgerät (2), aufweisend eine Kommunikationsschnittstelle (22), die derart eingerichtet ist, dass eine bidirektionale Kommunikation mit der Steuereinrichtung (3) erfolgen kann, wobei die Einrichtung (3) einerseits eine Softwareschicht zur Kommunikation mit dem Hörgerät (2) umfasst und andererseits dazu eingerichtet ist, eine Brücke zwischen der mit einer Software zur Fernsteuerung des Hörgeräts (2) ausgestatteten Steuerungsstation (4) und dem Hörgerät (2) zu bilden, und
- Mittel zur Schallisolierung (5) des Hörgeräts (2),
**dadurch gekennzeichnet, dass** es Bildaufnahmemittel umfasst, die derart bei der Prothese angeordnet sind, dass eine Visualisierung des Inneren des Ohrs erfolgen kann, wenn das Hörgerät (2) in dieses eingesetzt ist.

2. Fernhörtestsystem (1) nach Anspruch 1, **dadurch gekennzeichnet, dass** das Hörgerät (2) einen Hörgerätekörper (20) aufweist, der einem Eingangswandler zum Empfangen eines Eingangssignals und einen Mikrocontroller (21), der zum Steuern des Betriebs des Hörgeräts (2) konfiguriert ist, umfasst, wobei letzterer zudem einen elektroakustischen Ausgangswandler zum Erzeugen eines in Form von Tönen wahrnehmbaren Ausgangssignals und einen Signalübertragungskanal zum Übertragen von Signalen, die für den vom elektroakustischen Ausgangswandler zu erzeugenden oder von diesem kommenden Ton repräsentativ sind, umfasst.

3. Fernhörtestsystem (1) nach Anspruch 2, **dadurch gekennzeichnet, dass** die Bildaufnahmemittel am Ende des Signalübertragungskanals angeordnet sind.

4. Fernhörtestsystem (1) nach Anspruch 2 oder Anspruch 3, **dadurch gekennzeichnet, dass** der Signalübertragungskanal mit dem Hörgerätekörper (20) lösbar verbunden ist.

5. Fernhörtestsystem (1) nach einem der vorstehenden Ansprüche, **dadurch gekennzeichnet, dass** das Hörgerät (2) Schalldruckmessungsmittel aufweist.

6. Fernhörtestsystem (1) nach einem der vorstehenden Ansprüche, **dadurch gekennzeichnet, dass** die Schallisolierungsmittel in Form eines vorzugsweise aktiven Kopfhörers vorliegen.

7. Verfahren zum Erstellen eines Hörprofils einer Person mittels eines Fernhörtestsystems (1) nach einem der vorstehenden Ansprüche, wobei der Person zum Zwecke der Erstellung eines Hörprofils Hörgeräte angelegt werden, wobei den Hörgeräten Schallisolierungsmittel zugeordnet sind,
**dadurch gekennzeichnet, dass** es die folgenden Schritte umfasst:
- Übertragen von sich auf Tonsignalfolgen beziehende Daten über die Steuereinrichtung an das Hörgerät,
- Übertragen des Inhalts der Sprachantworten der Person über die Steuereinrichtung an die Steuerungsstation,
- kontinuierliches Messen des innerhalb der Schallisolierausrüstung herrschenden Schalldrucks und Übertragen der Daten über die Steuereinrichtung an die Steuerungsstation.

8. Verfahren zum Erstellen eines Hörprofils nach dem vorstehenden Anspruch, **dadurch gekennzeichnet, dass** die sich auf Tonsignalfolgen beziehenden Daten von der Steuerungsstation über die Steuereinrichtung an das Hörgerät übertragen werden.

9. Verfahren zum Erstellen eines Hörprofils nach Anspruch 7,
**dadurch gekennzeichnet, dass** die sich auf Tonsignalfolgen beziehenden Daten von der Steuereinrichtung als Reaktion auf eine Übertragungsgenehmigung der Steuerungsstation an das Hörgerät übertragen werden.

10. Verfahren zum Einstellen eines Hörgeräts (2), das eine bidirektionalen Kommunikationsschnittstelle aufweist, mittels eines Fernhörtestsystems (1) nach den Ansprüchen 1 bis 6, wobei das Verfahren einen Schritt des Übertragens von Daten zum Einstellen des Hörgeräts anhand eines von der Steuerungsstation über die Steuereinrichtung erstellten Hörprofils der Person umfasst.

11. Verfahren zum Einstellen eines Hörgeräts (2) nach Anspruch 10, **dadurch gekennzeichnet, dass** es einen Schritt des Auswählens einer Verwendungsumgebung aus einer Gruppe festgelegter Umgebungen, die in einem Untermodul einer auf die Steuereinrichtung heruntergeladen, dedizierten Anwendung gespeichert sind, umfasst.

12. Verfahren zum Einstellen eines Hörgeräts (2) nach Anspruch 11, **dadurch gekennzeichnet, dass** die Verwendungsumgebung mittels eines Algorithmus künstlicher Intelligenz unter Verwendung der von einem jeden Hörgeräteträger erfassten Verwendungsdaten ausgewählt wird.

13. Verfahren zum Einstellen eines Hörgeräts (2) nach Anspruch 11 oder 12, **dadurch gekennzeichnet, dass** die Verwendungsdaten und die ausgewählte Verwendungsumgebung erfasst und für Analysen und eine Optimierung nachfolgender personalisierter Feineinstellungen an eine auf der Steuerungsstation (4) gehosteten, persönliche Datenbank des Patienten übertragen werden.

14. Verfahren zum Stimulieren des Hörvermögens einer Person mittels eines Fernhörtestsystems (1) nach einem der Ansprüche 1 bis 6, umfassend einen Schritt des Erstellens eines Hörprofils der Person nach dem Verfahren nach einem der Ansprüche 7 bis 9.

## Claims

1. Remote hearing test system (1) comprising:
- a device (3) for controlling a hearing aid (2) comprising a communication interface arranged to allow two-way communication and software for playing predefined sequences of sounds stored in a memory module of the device in response to an instruction from a remote control station (4),
- at least one hearing aid (2) comprising a communication interface (22) arranged to allow two-way communication with the control device (3), said device (3) comprising a software layer for communicating with the hearing aid (2) and being arranged to provide a gateway between the control station (4), which is equipped with software for remote control of the hearing aid (2), and the hearing aid (2), and
- sound insulation means (5) for the hearing aid (2),
**characterized in that** it comprises image-capturing means that are arranged together with the hearing aid to allow the interior of the ear to be viewed when the ear is equipped with the hearing aid (2).

2. Remote hearing test system (1) according to claim 1,
**characterized in that** the hearing aid (2) comprises a hearing-aid body (20) comprising an input transducer for receiving an input signal and a microcontroller (21) configured to control the operation of the hearing aid (2), the hearing aid further comprising an electro-acoustic output transducer for producing a perceptible output signal in the form of sounds, and a signal transmission line for the transmission of signals representative of the sound to be produced by or coming from the electro-acoustic output transducer.

3. Remote hearing test system (1) according to claim 2,
**characterized in that** the image-capturing means are arranged at the end of the signal transmission line.

4. Remote hearing test system (1) according to claim 2 or claim 3, **characterized in that** the signal transmission line is removably connected to the hearing-aid body (20).

5. Remote hearing test system (1) according to any one of the preceding claims, **characterized in that** the hearing aid (2) comprises means for measuring the sound pressure.

6. Remote hearing test system (1) according to any one of the preceding claims, **characterized in that** the sound insulation means are in the form of a headset, preferably an active headset.

7. Method for establishing a hearing assessment of a person using a remote hearing test system (1) according to any one of the preceding claims, the person being equipped with hearing aids for the purpose of carrying out a hearing assessment, said hearing aids being associated with sound insulation means, **characterized in that** it comprises the steps of:
- transmitting data relating to sequences of sound signals to the hearing aid via the control device,
- transmitting the content of the voice responses of the person to the control station via the control device,
- continuously measuring the acoustic pressure present within the sound insulation equipment and transmitting the data to the control station via the control device.

8. Method for establishing a hearing assessment according to the preceding claim, **characterized in that** the data relating to sequences of sound signals are transmitted from the control station to the hearing aid via the control device.

9. Method for establishing a hearing assessment according to claim 7, **characterized in that** the data relating to sequences of sound signals are transmitted to the hearing aid by the control device in response to a transmission authorization from the control station.

10. Method for adjusting a hearing aid (2) comprising a two-way communication interface using a remote hearing test system (1) according to claims 1 to 6, the method comprising a step of transmitting data for adjusting the hearing aid on the basis of an established auditory profile of the person from the control station via the control device.

11. Method for adjusting a hearing aid (2) according to claim 10, **characterized in that** it comprises a step of selecting a usage environment from a set of specified environments stored in a sub-module of a dedicated application which is downloaded onto the control device.

12. Method for adjusting a hearing aid (2) according to claim 11, **characterized in that** the usage environment is selected using an artificial intelligence algorithm which utilizes the usage data collected from each wearer of the hearing aid.

13. Method for adjusting a hearing aid (2) according to claim 11 or claim 12, **characterized in that** the usage data and the selected usage environment are collected and transmitted into a database of personal information relating to the patient, which is hosted on the control station (4), for analysis and optimization of subsequent customized fine adjustments.

14. Method for stimulating the hearing capability of a person using a remote hearing test system (1) according to any one of claims 1 to 6, comprising a step of establishing a hearing assessment for the person according to the method according to any one of claims 7 to 9.
